# EUROPEAN PATENT APPLICATION

(11) **EP 4 420 621 A1**
(43) Date of publication of application: **28.08.2024**
(21) Application number: 23889844.9
(22) Date of filing: 30.05.2023
(51) Int. Cl.: A61B 17/22

(54) **CUTTING GUIDE WIRE AND THROMBUS ASPIRATION SYSTEM**

(30) Priority: 17.11.2022 CN 202211439559
(71) Applicant: Shanghai Int Medical Instruments Co., Ltd., Shanghai 201800 (CN)
(72) Inventor: ZHOU, Hongxin, Shanghai 201800 (CN); LIANG, Dongke, Shanghai 201800 (CN); HUAN, Wei, Shanghai 201800 (CN); SUN, Chunming, Shanghai 201800 (CN); CHENG, Guang, Shanghai 201800 (CN)
(74) Representative: J A Kemp LLP
(86) International application number: PCT/CN2023/097174
(87) International publication number: WO 2024/103686

(57) **Abstract**

The present application provides a cutting guide wire and a thrombus suction system. The cutting guide wire includes a guide wire body and a thrombus cutting member, a distal end of the guide wire body is suitable for being arranged in a suction catheter in a passing-through mode, the thrombus cutting member is connected to the distal end of the guide wire body and suitable for being placed in the suction catheter, and the thrombus cutting member has a cutting state of moving in the suction catheter to cut a thrombus in the suction catheter under a driving action of the guide wire body; wherein the thrombus cutting member includes a cutting portion, and a cross-sectional area of the cutting portion gradually increases in a direction from a distal end thereof to a proximal end thereof. The thrombus cutting member is connected to the distal end of the guide wire body and includes the cutting portion, the cross-sectional area of the cutting portion gradually increases in the direction from the distal end thereof to the proximal end thereof, so when the thrombus cutting member is placed in the suction catheter to move so as to cut the thrombus in the suction catheter through the guide wire body, the cutting portion in a tip shape can cut and crush the thrombus blocking the suction catheter, and an interior of the suction catheter can be prevented from being blocked.

## Description

### CROSS-REFERENCE TO RELATED APPLICATIONS

The present application claims priority to Chinese Patent Application No. 202211439559 .5, filed to the China Patent Office on November 17, 2022 and entitled "CUTTING GUIDE WIRE AND THROMBUS SUCTION SYSTEM", the entire content of which is incorporated herein by reference.

### TECHNICAL FIELD

The present application relates to the technical field of medical equipment, in particular to a cutting guide wire and a thrombus suction system.

### BACKGROUND

Thrombus aspiration is an effective method in clinical practice in recent years and is intended to rapidly suck out a thrombus blocking a diseased blood vessel under a mechanical action, which can relieve a thrombus load and microvascular embolization, change coronary inflow and reduce probability of occurrence of postoperation "slow flow" and "no-reflow".

A thrombus suction catheter system in the prior art includes a negative pressure suction pump, a thrombus suction catheter, an internal tube, a Y-type connector and a guide wire. In use, the guide wire is delivered into a diseased region of a human body, then the thrombus suction catheter and the internal tube are inserted into the diseased region along a tail end of the super-smooth guide wire, the negative pressure suction pump communicates with the thrombus suction catheter through the Y-type connector, the guide wire and the internal tube are withdrawn, then a negative pressure is applied to the thrombus suction catheter through a negative pressure suction apparatus, an end of the catheter is targeted at the thrombus, and thus an effect of sucking the thrombus out of the human body from a blood vessel through the thrombus suction catheter is achieved.

However, the above thrombus suction catheter system can suck a fresh and soft embolus and an acute thrombus block, but is prone to blocking the catheter while sucking a subacute thrombus, which then affects suction of the thrombus by the thrombus suction catheter.

### SUMMARY OF THE INVENTION

Thus, a technical problem to be solved by the present application is that the thrombus suction catheter system in the prior art can suck a fresh and soft embolus and an acute thrombus block, but is prone to blocking a catheter while sucking a subacute thrombus, which then affects suction of the thrombus by the thrombus suction catheter.

Thus, the present application provides a cutting guide wire, including:
a guide wire body, with a distal end of the guide wire body configured to be arranged in a suction catheter in a passing-through mode; and
a thrombus cutting member, connected to the distal end of the guide wire body, the thrombus cutting member being configured to be placed in the suction catheter, and the thrombus cutting member having a cutting state of moving in the suction catheter to cut a thrombus in the suction catheter under a driving action of the guide wire body;
wherein the thrombus cutting member includes a cutting portion, and a cross-sectional area of the cutting portion gradually increases in a direction from a distal end thereof to a proximal end thereof.

Optionally, a lateral wall surface of the cutting portion is a circular truncated cone surface or a paraboloid surface.

Optionally, a plurality of protrusions and/or recesses are arranged on a peripheral side surface of the cutting portion.

Optionally, the thrombus cutting member further includes a connecting portion, and the connecting portion is connected between the guide wire body and the cutting portion.

Optionally, a cross-sectional area of the connecting portion gradually increases in a direction from a proximal end thereof to a distal end thereof.

Optionally, the connecting portion is of a circular truncated cone structure or a paraboloid structure.

Optionally, the thrombus cutting member further includes a spiral portion, the spiral portion is connected to the connecting portion, and the guide wire body is arranged inside the spiral portion in a passing-through mode.

Optionally, the cutting guide wire further includes a rotary knob connected to a proximal end of the guide wire body so as to be able to drive the guide wire body to rotate.

A thrombus suction system includes the above cutting guide wire.

Optionally, the thrombus suction system further includes a negative pressure source, a connector, the cutting guide wire, a suction catheter and a catheter handle, the connector is connected between the negative pressure source and the suction catheter, the catheter handle is connected to a proximal end of the suction catheter, the cutting guide wire is suitable for being placed in the suction catheter, and a plurality of lateral holes are formed in a lateral wall of a distal end of the suction catheter.

The cutting guide wire and the thrombus suction system provided by the present application have the following advantages.
1. The cutting guide wire provided by the present application includes the guide wire body and the thrombus cutting member, the distal end of the guide wire body is suitable for being arranged in the suction catheter in a passing-through mode, the thrombus cutting member is connected to the distal end of the guide wire body, the thrombus cutting member is suitable for being placed in the suction catheter, and the thrombus cutting member has the cutting state of moving in the suction catheter to cut the thrombus in the suction catheter under a driving action of the guide wire body; wherein the thrombus cutting member includes the cutting portion, and the cross-sectional area of the cutting portion gradually increases in the direction from the distal end thereof to the proximal end thereof.
2. According to the cutting guide wire provided by the present application, the thrombus cutting member further includes the spiral portion, the spiral portion is connected to the connecting portion, and the guide wire body is arranged inside the spiral portion in a passing-through mode.
   In the cutting guide wire of this structure, by arranging the spiral portion on the connecting portion, during cleaning of the thrombus, the whole spiral portion is inserted into the thrombus to cut and crush the thrombus, then the spiral portion may bring the crushed thrombus out of the thrombus block while being pulled out from the thrombus, and thus an effect of cleaning the thrombus can be improved.

3. The thrombus suction system provided by the present application further includes the negative pressure source, the connector, the cutting guide wire, the suction catheter and the catheter handle, the connector is connected between the negative pressure source and the suction catheter, the catheter handle is connected to the proximal end of the suction catheter, the cutting guide wire is suitable for being placed in the suction catheter, and the plurality of lateral holes are formed in the lateral wall of the distal end of the suction catheter.

In the thrombus suction system of this structure, by forming the plurality of lateral holes in the lateral wall of the distal end of the suction catheter, the thrombus on a lateral wall in a blood vessel can be cleaned by using the thrombus suction catheter which is much smaller than an inner diameter of the blood vessel. Besides, if an opening of a head end or one of lateral holes of the suction catheter is blocked by the thrombus, other openings may continue sucking the thrombus without replacing the catheter, the thrombus is sucked completely and rapidly, and a blood loss volume is reduced.

### BRIEF DESCRIPTION OF THE DRAWINGS

In order to more clearly describe specific implementations of the present application or technical solutions in the prior art, the accompanying drawings needed by the description in the specific implementations or in the prior art will be briefly introduced below. Apparently, the accompanying drawings in the following description are some implementations of the present application. Those ordinarily skilled in the art may also obtain other accompanying drawings according to these accompanying drawings without making creative work.
Fig. 1 is a schematic view of a whole structure of a cutting guide wire and a thrombus suction system provided by an embodiment of the present application.
Fig. 2 is a schematic structural diagram of a cutting guide wire and a catheter handle in a thrombus suction system provided by an embodiment of the present application.
Fig. 3 is a schematic structural explosive view of a cutting guide wire and a catheter handle in a thrombus suction system provided by an embodiment of the present application.
Fig. 4 is a schematic structural diagram of a distal end of a cutting guide wire and a thrombus suction system provided by an embodiment of the present application.
Fig. 5 is a schematic structural explosive view of a distal end of a cutting guide wire and a thrombus suction system provided by an embodiment of the present application.
Fig. 6 is a schematic structural diagram of a thrombus cutting member in a cutting guide wire and a thrombus suction system provided by an embodiment of the present application.
Fig. 7 is another schematic structural diagram of a thrombus cutting member in a cutting guide wire and a thrombus suction system provided by an embodiment of the present application.

### Descriptions of reference numerals:

1, guide wire body;
2, thrombus cutting member; 21, cutting portion; 22, connecting portion; 23, spiral portion;
3, suction catheter; 31, lateral hole;
4, catheter handle;
5, lining; and
6, protecting sleeve.

### DETAILED DESCRIPTION

The technical solutions of the present application will be clearly and completely described in the following with reference to the accompanying drawings. Apparently, the described embodiments are some rather than all of the embodiments of the present application. All other embodiments obtained by those ordinarily skilled in the art based on the embodiments of the present application without making creative efforts fall within the protection scope of the present application.

In the description of the present application, it needs to be noted that directions or position relations indicated by terms such as "center", "upper", "lower", "left", "right", "vertical", "horizontal", "inner" and "outer" are directions or position relations as shown in the accompanying drawings and are only intended to conveniently describe the present application and simplify the description but not to indicate or imply that an apparatus or element referred to necessarily has a specific direction or is constructed or operated in a specific direction, so as not to be understood as a limitation on the present application. Besides, terms such as "first", "second" and "third" are only used for the description instead of being understood as indicating or implying a relative significance. Besides, technical features involved in different implementations of the present application described below may be combined mutually as long as they do not constitute a conflict with one another.

In the description of the present application, it needs to be noted that a distal end in the present application is an end close to a thrombus cutting member, and a proximal end is an end close to a catheter handle.

### Embodiment 1

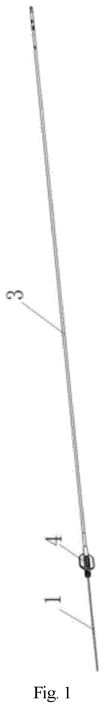

This embodiment provides a cutting guide wire, as shown in Fig. 1 to Fig. 7, including a guide wire body 1 and a thrombus cutting member 2, the thrombus cutting member 2 is connected to a distal end of the guide wire body 1, the distal end of the guide wire body 1 and the thrombus cutting member 2 are used for being arranged in a suction catheter 3 in a passing-through mode, the thrombus cutting member 2 is driven to move back and forth in the suction catheter 3 by hand-holding a proximal end of the guide wire body 1 so as to cut a thrombus in the suction catheter 3, and a situation that the suction catheter 3 is blocked and suction of the thrombus by the thrombus suction catheter 3 is affected is prevented.

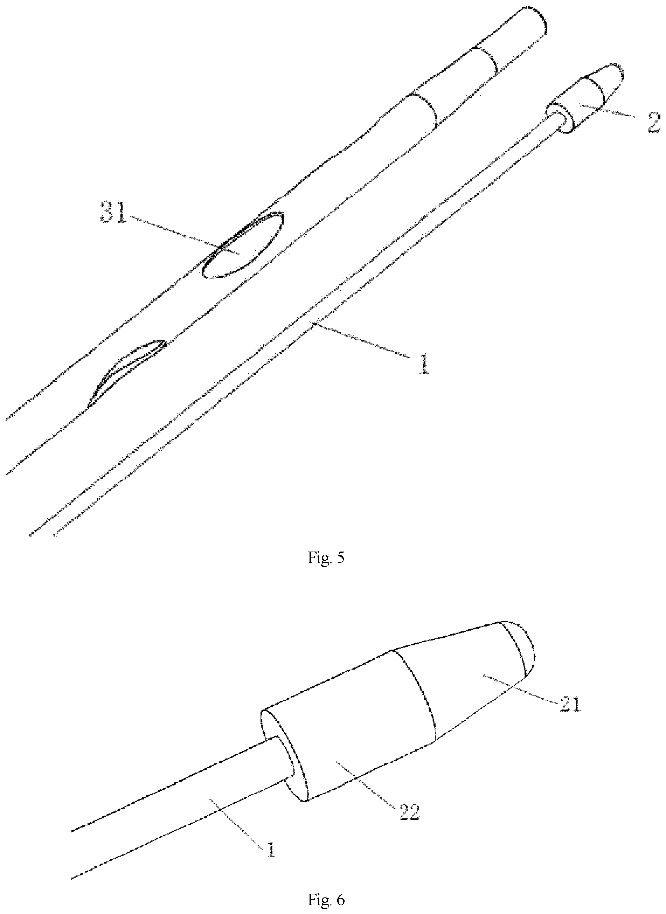

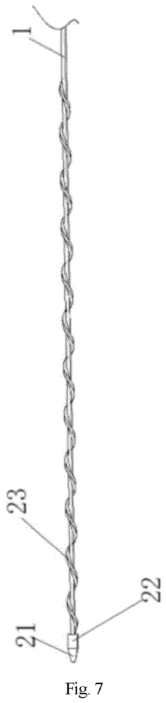

As shown in Fig. 6 and Fig. 7, the thrombus cutting member 2 includes a cutting portion 21 and a connecting portion 22, and the connecting portion 22 is connected between the guide wire body 1 and the cutting portion 21. A cross-sectional area of the cutting portion 21 gradually increases in a direction from a distal end thereof to a proximal end thereof, namely, the cutting portion 21 is of a circular truncated cone structure or a circular cone structure or a paraboloid structure.

It may be understood that in some other implementations, the cutting portion 21 may also be of another tip-shaped structure, and the tip-shaped structure here can be more easily inserted into the thrombus while the cutting portion 21 touches the thrombus so as to cut and crush the thrombus.

It can be understood that in some other implementations, a plurality of protrusions which can improve the effect of cutting the thrombus or a plurality of recesses may be arranged on a surface of the cutting portion 21.

As shown in Fig. 6, the connecting portion 22 is columnar, and the columnar structure can make the thrombus cutting member 2 conveniently move in the thrombus.

In some other implementations, a cross-sectional area of the connecting portion 22 gradually increases in a direction from a proximal end thereof to a distal end thereof, namely, the connecting portion 22 is of a circular truncated cone structure or a paraboloid structure.

In some other implementations, the thrombus cutting member 2 further includes a spiral portion 23, the spiral portion 23 is fixedly connected to the connecting portion 22, the guide wire body 1 is arranged inside the spiral portion 23 in a passing-through mode, during cleaning of the thrombus, the spiral portion 23 is inserted into the thrombus to cut and crush the thrombus, and then the spiral portion 23 may bring the crushed thrombus out of the thrombus block while being pulled out from the thrombus, so that the effect of cleaning the thrombus can be improved.

In some other implementations, a rotary knob is connected to a proximal end of the guide wire body 1, and an operator may control the guide wire body 1 to rotate through the rotary knob.

The guide wire body 1 is made of a nickel-titanium alloy material which is good in shape memory, and a surface of the distal end of the guide wire body 1 contains a hydrophilic coating, so that the guide wire body 1 is good in controllability.

In some other implementations, the guide wire body 1 and the thrombus cutting member 2 may be arranged at an included angle in a connection position, namely, the included angle exists between an axis of the guide wire body 1 and an axis of the thrombus cutting member 2, so that the bent thrombus cutting member 2 can be used for stripping the thrombus off a blood vessel wall while crushing the thrombus, which is better for thrombus suction.

### Embodiment 2

This embodiment provides a thrombus suction system, as shown in Fig. 1 to Fig. 7, including a suction catheter 3, a catheter handle 4, a lining 5, a protecting sleeve 6 and a cutting guide wire, and the cutting guide wire is the cutting guide wire in Embodiment 1.

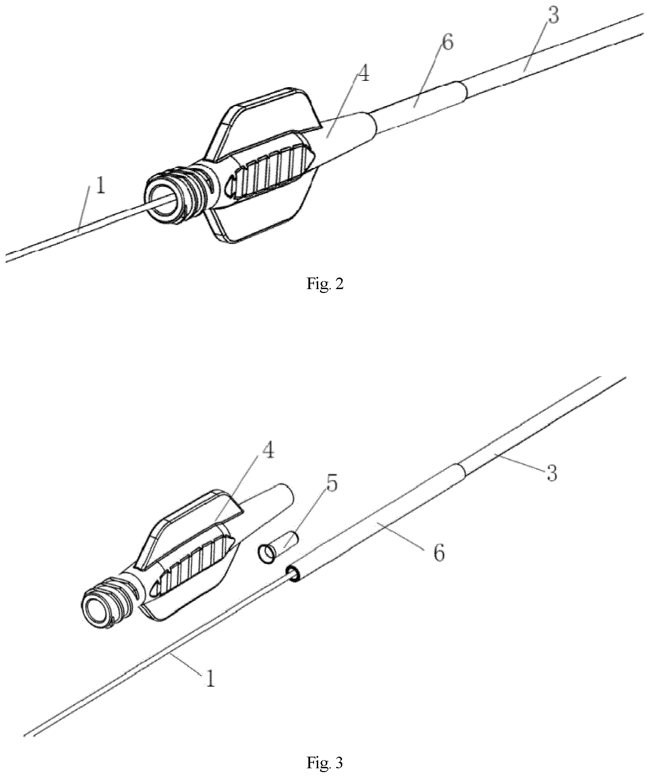

As shown in Fig. 2 and Fig. 3, the catheter handle 4 is connected to the protecting sleeve 6 in an inserted mode, the protecting sleeve 6 is connected to the suction catheter 3 in an inserted mode through the lining 5, a distal end of the cutting guide wire is inserted into the suction catheter 3, and the catheter handle 4 is used for controlling the catheter.

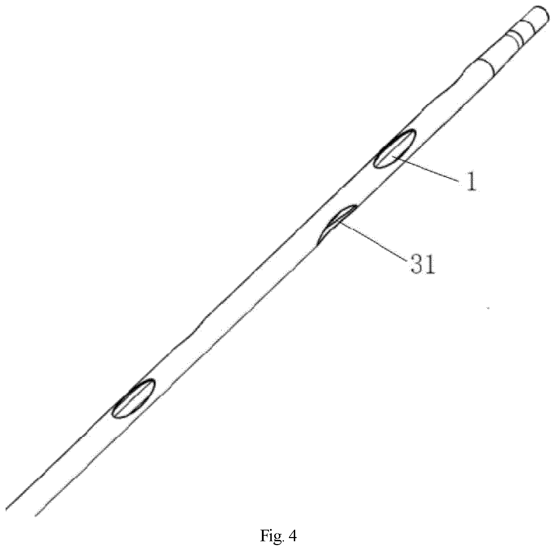

As shown in Fig. 4 and Fig. 5, a plurality of lateral holes 31 are formed in a distal end of the suction catheter 3, and by forming the lateral holes 31 in a lateral wall of the distal end of the suction catheter 3, the thrombus on a lateral wall in a blood vessel can be cleaned by using the thrombus suction catheter 3 which is much smaller than an inner diameter of the blood vessel. Besides, if an opening of a head end or one of lateral holes 31 of the suction catheter 3 is blocked by the thrombus, other openings may continue sucking the thrombus without replacing the catheter, the thrombus is sucked completely and rapidly, and a blood loss volume is reduced.

It can be understood that the number of the lateral holes 31 may be determined according to a size of the suction catheter 3 or a use demand, for example, two, three, five, eight, etc., and preferably, the lateral holes 31 are formed in different sides of the suction catheter 3, so as to implement sucking thrombi in a larger range.

The suction catheter 3 adopts a three-layer composite cladding technology, an inner layer is a PTFE layer which is small in resistance and facilitates passing of the thrombus, a middle layer is of a stainless steel braided structure which is high in supporting, good in pushing and resistant to bending, and an outer layer is Pebax resin which is good in biocompatibility. Two ends of each lateral hole 31 have developing Marker points which can indicate positions of the lateral holes 31, and a head end of the suction catheter 3 is provided with a head end developing segment which can indicate a position of the head end.

In some other implementations, the thrombus suction system further includes the negative pressure source and the connector, the connector is connected between the negative pressure source and the suction catheter 3, the negative pressure source is a negative pressure suction pump, the connector is a rotary or press-type Y valve, and blood oozing can be effectively prevented.

A working flow of the thrombus suction system provided by this embodiment is as follows:
first, the cutting guide wire enters a diseased region of a human body along a related instrument, then the suction catheter 3 is inserted into the diseased region along a tail end of the cutting guide wire, the negative pressure suction pump is connected to a side branch of the Y-type connector to be screwed up, the cutting guide wire is withdrawn, then a negative pressure is applied through the negative pressure suction pump and stabilized, the catheter is slowly pushed and rotated after the pressure is stable, the lateral holes 31 of the catheter are targeted at the thrombus, the thrombus is sucked out of the body from a blood vessel, and in the case that the catheter is blocked, the thrombus is cut by using the matched cutting guide wire and the thrombus is cut and sucked at the same time.

Apparently, the above embodiments are merely examples made for clear description but not for limiting the implementations. Those ordinarily skilled in the art can also make modifications or variations in other different forms based on the above description. All implementations do not need to be and cannot be exhaustively cited here. Apparent modifications or variations derived from this still fall within the protection scope of the present application.

## Claims

1. A cutting guide wire, comprising:
a guide wire body (1), with a distal end of the guide wire body (1) configured to be arranged in a suction catheter (3) in a passing-through mode; and
a thrombus cutting member (2), connected to the distal end of the guide wire body (1), the thrombus cutting member (2) being configured to be placed in the suction catheter (3), and the thrombus cutting member (2) having a cutting state of moving in the suction catheter (3) to cut a thrombus in the suction catheter (3) under a driving action of the guide wire body (1);
wherein the thrombus cutting member (2) comprises a cutting portion (21), and a cross-sectional area of the cutting portion (21) gradually increases in a direction from a distal end thereof to a proximal end thereof.

2. The cutting guide wire according to claim 1, wherein a lateral wall surface of the cutting portion (21) is a circular truncated cone surface or a paraboloid surface.

3. The cutting guide wire according to claim 1 or 2, wherein a plurality of protrusions and/or recesses are arranged on a peripheral side surface of the cutting portion (21).

4. The cutting guide wire according to claim 3, wherein the thrombus cutting member (2) further comprises a connecting portion (22), and the connecting portion (22) is connected between the guide wire body (1) and the cutting portion (21).

5. The cutting guide wire according to claim 4, wherein a cross-sectional area of the connecting portion (22) gradually increases in a direction from a proximal end thereof to a distal end thereof.

6. The cutting guide wire according to claim 5, wherein the connecting portion (22) is of a circular truncated cone structure or a paraboloid structure.

7. The cutting guide wire according to claim 3, wherein the thrombus cutting member (2) further comprises a spiral portion (23), the spiral portion (23) is connected to the connecting portion (22), and the guide wire body (1) is arranged inside the spiral portion (23) in a passing-through mode.

8. The cutting guide wire according to claim 7, further comprising a rotary knob connected to a proximal end of the guide wire body (1) so as to be able to drive the guide wire body (1) to rotate.

9. A thrombus suction system, comprising the cutting guide wire according to any one of claims 1-8.

10. The thrombus suction system according to claim 9, comprising a negative pressure source, a connector, the cutting guide wire, a suction catheter (3) and a catheter handle (4), the connector being connected between the negative pressure source and the suction catheter (3), the catheter handle (4) being connected to a proximal end of the suction catheter (3), the cutting guide wire configured to be placed in the suction catheter (3), and a plurality of lateral holes (31) are formed in a lateral wall of a distal end of the suction catheter (3).
